# EUROPEAN PATENT SPECIFICATION

(11) **EP 3 177 293 B1**
(45) Date of publication and mention of the grant of the patent: **05.05.2021**
(21) Application number: 15711272.3
(22) Date of filing: 10.03.2015
(51) Int. Cl.: A61K 31/4995, A61P 17/08

(54) **METHOD OF TREATING SKIN CONDITIONS**
VERFAHREN ZUR BEHANDLUNG VON HAUTKRANKHEITEN
MÉTHODE DE TRAITEMENT DE TROUBLES CUTANÉS

(30) Priority: 12.03.2014 US 201461951604 P
(43) Date of publication of application: 14.06.2017
(73) Proprietor: Astrazeneca AB, 151 85 Södertälje (SE)
(72) Inventor: BUCKETT, Linda, Karen, Macclesfield Cheshire SK10 4TG (GB); FLOETTMANN, Jan, Eike, Macclesfield Cheshire SK10 4TG (GB); JONES, Huw, Macclesfield Cheshire SK10 4TG (GB); TURNBULL, Andrew, SE-431 83 Molndal (SE)
(74) Representative: HGF
(86) International application number: PCT/GB2015/050704
(87) International publication number: WO 2015/136275

(56) References cited:
- WO-A1-2009/081195
- WO-A2-2006/023654
- JONAS G. BARLIND ET AL: "Design and Optimization of Pyrazinecarboxamide-Based Inhibitors of Diacylglycerol Acyltransferase 1 (DGAT1) Leading to a Clinical Candidate Dimethylpyrazinecarboxamide Phenylcyclohexylacetic Acid (AZD7687)", JOURNAL OF MEDICINAL CHEMISTRY, vol. 55, no. 23, 13 December 2012 (2012-12-13), pages 10610-10629, XP055077223, ISSN: 0022-2623, DOI: 10.1021/jm301296t
- SATO TAKASHI ET AL: "Adapalene suppresses sebum accumulationviathe inhibition of triacylglycerol biosynthesis and perilipin expression in differentiated hamster sebocytesin vitro", JOURNAL OF DERMATOLOGICAL SCIENCE, ELSEVIER SCIENCE PUBLISHERS, SHANNON, IE, vol. 70, no. 3, 14 February 2013 (2013-02-14), pages 204-210, XP028543636, ISSN: 0923-1811, DOI: 10.1016/J.JDERMSCI.2013.02.003
- Eric S. Muise ET AL: "Identification and Characterization of Sebaceous Gland Atrophy-Sparing DGAT1 Inhibitors", PLoS ONE, vol. 9, no. 2, 1 January 2014 (2014-01-01) , page e88908, XP055597100, DOI: 10.1371/journal.pone.0088908

## Description

### Background of the Invention

The sebaceous glands are microscopic glands in the skin that secrete sebum, a mixture of waxes, fatty acids, di- and tri-glycerides, squalene and cholesterol. These glands are found on all surfaces of the body, except the palms and the soles of the feet. A number of medical conditions are associated with sebaceous glands, including acne, seborrheic cysts, sebaceous hyperplasia, seborrhea, seborrheic dermatitis, sebaceous adenomas and seborrhea and sebaceoma. Accordingly, there is a need for new therapies that can target these disorders. Eric S. Muise et al ("Identification and Characterisation of sebaceous gland atrophy-sparing, DGAT1 Inhibitors", PLoS ONE, vol, 9, no.2, 1 January 2014, page e88908), disclosed that DGAT 1^{-/-} mice develop leptin-dependent abnormal skin phenotypes, characterised by sebaceous gland atrophy and hair loss.

### Summary of the Invention

In a first aspect of the present invention, there is provided an effective amount of 2-((1R,4R)-4-(4-(6-carbamoyl-3,5-dimethylpyrazin-2-yl)phenyl)cyclohexyl)acetic acid, or a pharmaceutically acceptable salt thereof for use in, treating a disorder associated with sebaceous glands in a subject.

In some embodiments, the disorder is selected from acne, seborrhea, sebaceoma, sebaceous carcinoma, sebborheic dermatitis, sebborheic cysts, sebaceous adenoma and sebaceous hyperplasia.

In a further aspect of the present, there is provided an effective amount of 2-((1R,4R)-4-(4-(6-carbamoyl-3,5-dimethylpyrazin-2-yl)phenyl)cyclohexyl)acetic acid, or a pharmaceutically acceptable salt thereof, for use in inducing sebaceous gland atrophy in a subject.

In a further aspect of the present invention, there is provided an effective amount of 2-((1*R*,4*R*)-4-(4-(6-carbamoyl-3,5-dimethylpyrazin-2-yl)phenyl)cyclohexyl)acetic acid, or a pharmaceutically acceptable salt thereof, for use in reducing sebum production in a subject.

### Brief Description of the Drawings

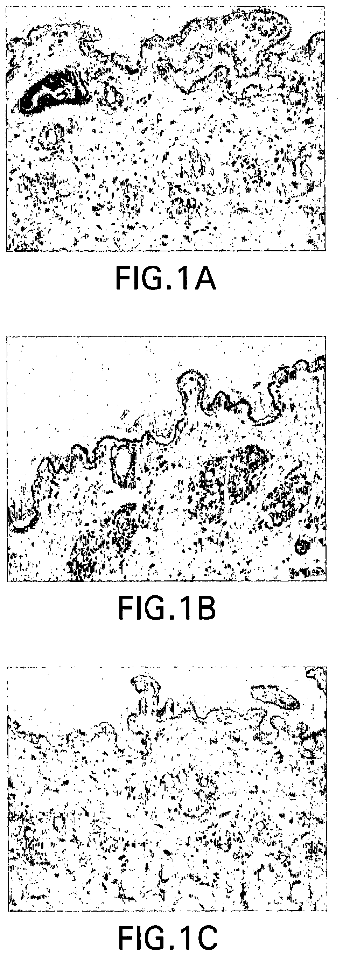

**Figure 1** illustrates photomicrographs of skin from (A) a control mouse, (B) a mouse dosed with 300 mg/kg/day for 28 days and (C) a mouse dosed with 300 mg/kg/day 2-((1*R*,4*R*)-4-(4-(6-carbamoyl-3,5-dimethylpyrazin-2-yl)phenyl)cyclohexyl)acetic acid for 28 days followed by a drug free recovery period of 28 days. Note in (A) and (C) the normal appearance of sebum filled sebaceous glands and the severely atrophic sebaceous glands with substantially reduced sebum in (B). (Haematoxylin and eosin. Original objective lens magnifications x 20) 196x435mm (300 x 300 DPI).

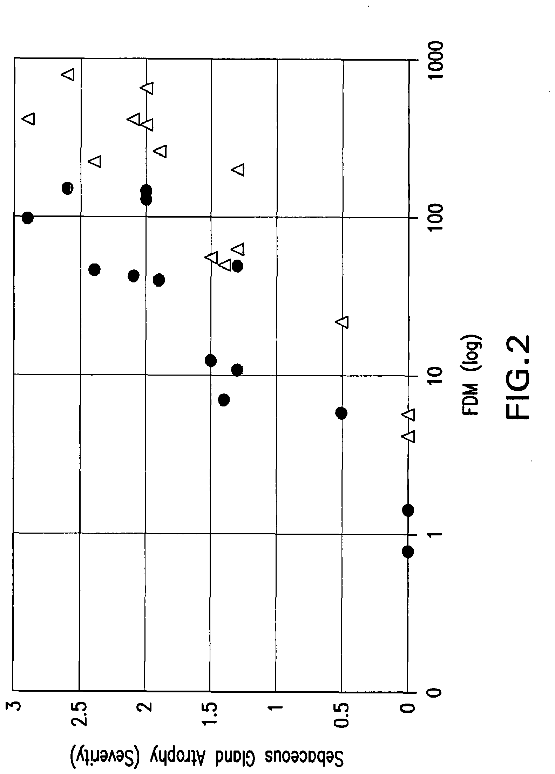

**Figure 2** illustrates the effect of different degrees of 2-((1*R*,4*R*)-4-(4-(6-carbamoyl-3,5-dimethylpyrazin-2-yl)phenyl)cyclohexyl)acetic acid-mediated DGAT1 inhibition, expressed as FDM (log), on sebaceous gland atrophy at Cmax (grey triangles) and Cmin (black circles) in mice. A dose-related increase in sebaceous gland atrophy is demonstrated. The score for severity of sebaceous gland atrophy is 0 (absent), 1 (minimal), 2 (mild), and 3 (moderate). Data are group means for males and for females (n≥10/sex/group for histopathology data and n=3/sex/group for exposure data) and are taken from the 1 and 6 months studies.

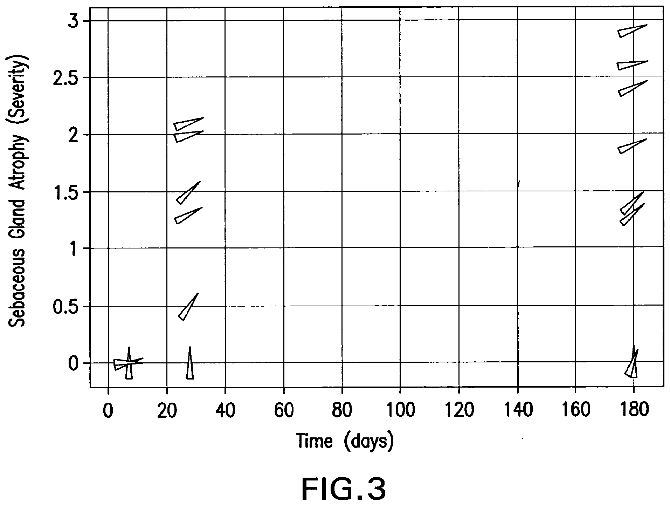

**Figure 3** illustrates Effects of duration of 2-((1*R*,4*R*)-4-(4-(6-carbamoyl-3,5-dimethylpyrazin-2-yl)phenyl)cyclohexyl)acetic acid dosing and different levels of DGAT1 inhibition, expressed as FDM, on sebaceous gland atrophy in mice. A time- and dose-related increase in sebaceous gland atrophy is demonstrated. Data points (grey triangles) are rotated to the right by increasing level of DGAT inhibition. The score for severity of sebaceous gland atrophy is 0 (absent), 1 (minimal), 2 (mild), and 3 (moderate). Data are group means for males and females (n≥10/sex/group for histopathology data and n=3/sex/group for exposure data at Cmax). 63x45mm (600 x 600 DPI).

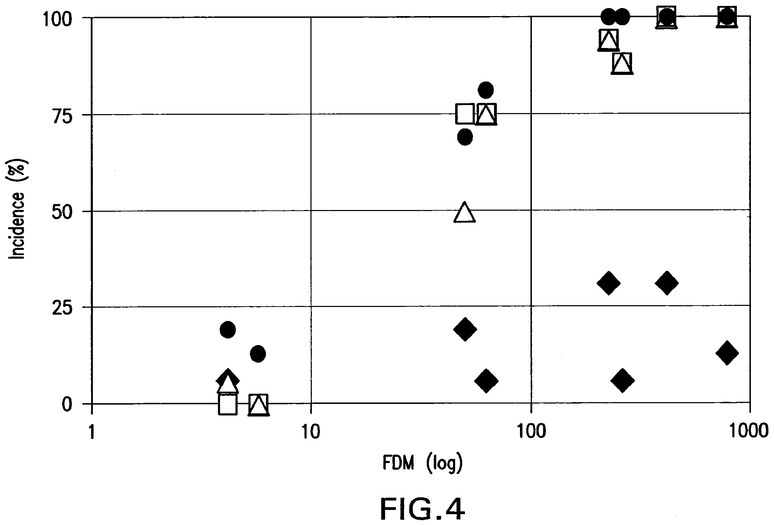

**Figure 4** illustrates the effect of different degrees of DGAT1 inhibition, expressed as FDM, on sebaceous gland atrophy (red squares), epidermal hyperplasia/hyperkeratosis (green triangles), and in-life observations of hair loss (black circles) and skin lesions (yellow diamonds) in mice dosed with 2-((1*R*,4*R*)-4-(4-(6-carbamoyl-3,5-dimethylpyrazin-2-yl)phenyl)cyclohexyl)acetic acid for 6 months. Data are group means for males and females (n≥10/sex/group for histopathology data and n=3/sex/group for exposure data). 63x45mm (600 x 600 DPI).

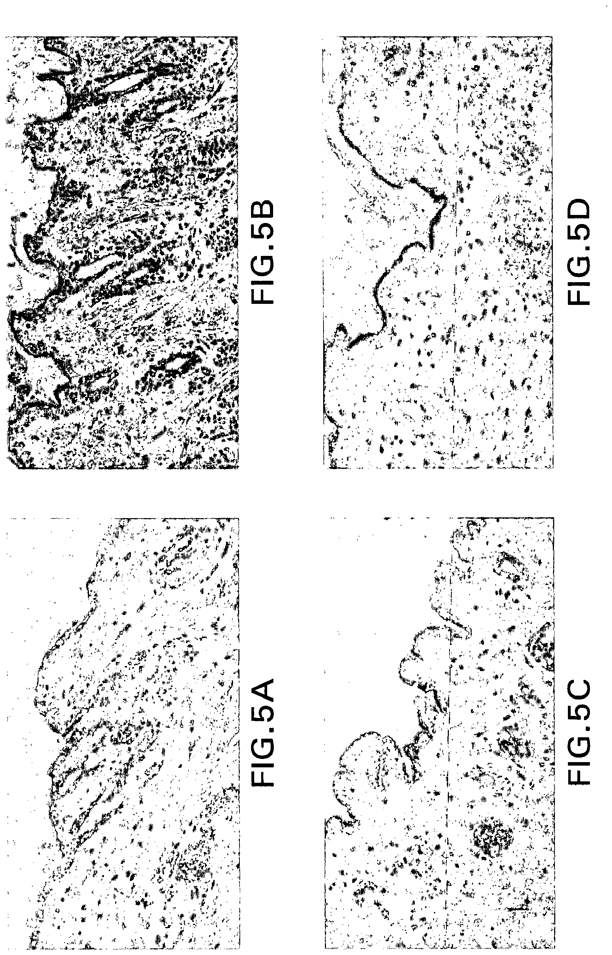

**Figure 5** illustrates photomicrographs of skin from (A) a male control mouse, (B) a male mouse dosed with 300 mg/kg/day 2-((1*R*,4*R*)-4-(4-(6-carbamoyl-3,5-dimethylpyrazin-2-yl)phenyl)cyclohexyl)acetic acid for 6 months, (C) a male DGAT wildtype mouse, and (D) a male homozygous DGAT1 knock out (-/-) mouse. Note in (A) and (C) the normal appearance of sebum filled sebaceous glands and in (B) and (D) the substantial atrophy of sebaceous glands with much reduced sebum and mild hyperkeratosis. (Haematoxylin and eosin. Original objective lens magnifications x 20). 181x104mm (300 x 300 DPI)

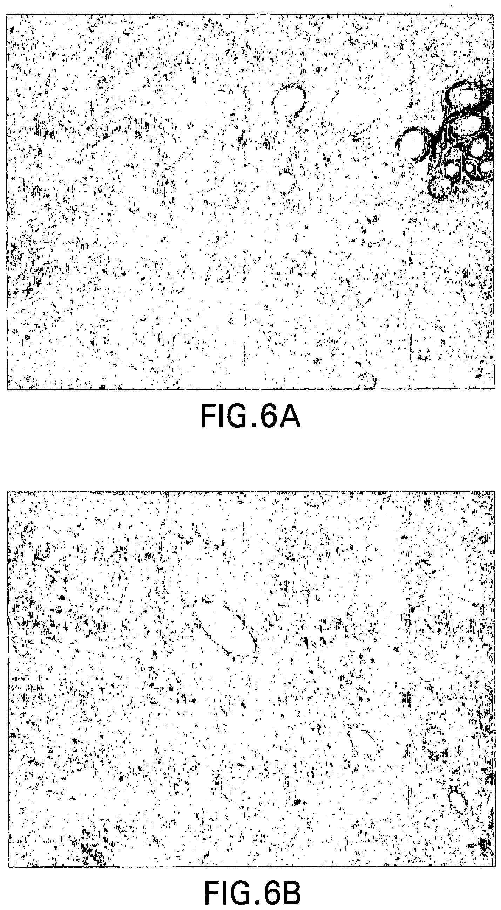

**Figure 6** illustrates photomicrographs of skin of (A) a control dog and (B) a dog dosed with 100 mg/kg/day 2-((1*R*,4*R*)-4-(4-(6-carbamoyl-3,5-dimethylpyrazin-2-yl)phenyl)cyclohexyl)acetic acid for 6 months. Note in (A) the prominent sebum filled sebaceous glands and in (B) the atrophic sebaceous glands which contain little sebum. (Haematoxylin and eosin. Original objective lens magnifications x 20) 130x190mm (300 x 300 DPI.

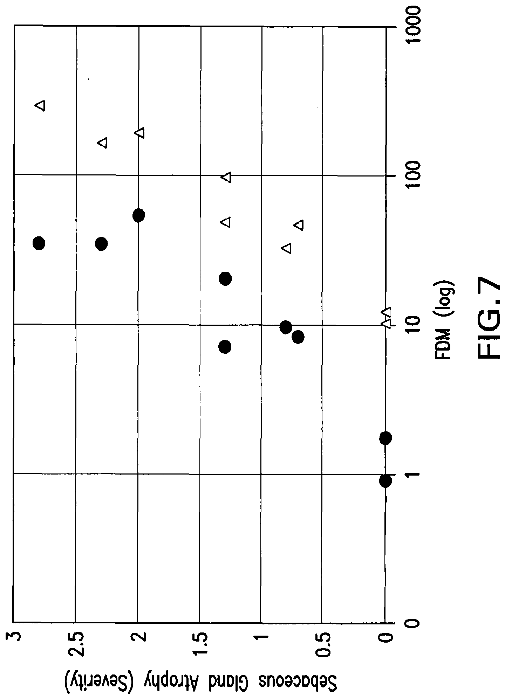

**Figure 7** illustrates the effect of different degrees of 2-((1R,4R)-4-(4-(6-carbamoyl-3,5-dimethylpyrazin-2-yl)phenyl)cyclohexyl)acetic acid-mediated DGAT1 inhibition, expressed as FDM (log), on sebaceous gland atrophy at Cmax (grey triangles) and Cmin (black circles) in dogs. A dose-related increase in sebaceous gland atrophy is demonstrated. The score for severity of sebaceous gland atrophy is 0 (absent), 1 (minimal), 2 (mild), and 3 (moderate). Data are combined group means for males and females (n=4/sex/group for histopathology data and exposure data) and are taken from the 14 day and 6 months studies. 63x45mm (600 x 600 DPI).

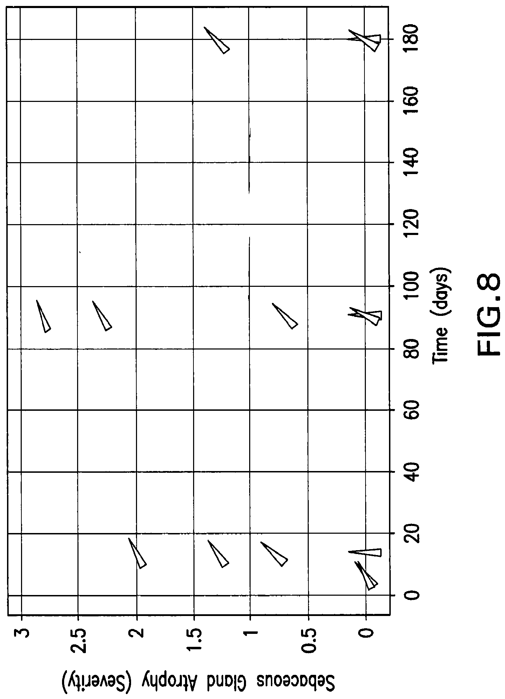

**Figure 8** illustrates the effects of duration of 2-((1R,4R)-4-(4-(6-carbamoyl-3,5-dimethylpyrazin-2-yl)phenyl)cyclohexyl)acetic acid dosing and different degrees of DGAT1 inhibition, expressed as FDM, on sebaceous gland atrophy in dogs. A time- and dose-related increase in sebaceous gland atrophy is demonstrated. Data points (grey triangles) are rotated to the right by increasing level of DGAT1 inhibition. The score for severity of sebaceous gland atrophy is 0 (absent), 1 (minimal), 2 (mild), and 3 (moderate). Data are combined group means for males and females (n=4/sex/group for histopathology data and exposure data at Cmax). 63x45mm (600 x 600 DPI).

### Detailed Description of the Invention

The invention pertains, in the first aspect, to an effective amount of 2-((1*R*,4*R*)-4-(4-(6-carbamoyl-3,5-dimethylpyrazin-2-yl)phenyl)cyclohexyl)acetic acid, or a pharmaceutically acceptable salt thereof, for use in treating a disorder associated with sebaceous glands.

The language "treat," "treating" and "treatment" includes the reduction or inhibition of enzyme or protein activity related to sebaceous glands in a subject, amelioration of one or more symptoms of a disorder associated with sebaceous glands in a subject, or the slowing or delaying of progression of a disorder associated with sebaceous glands in a subject.

The language "sebaceous gland" includes unilobular or multilobular glands that secrete sebum. Sebaceous glands include pilosebaceous units, fordyce spots, meibomian glands, glands of the Zeiss and Montgomery areolar tubercles.

The language "disorder associated with sebaceous glands" includes diseases, conditions and symptoms related to sebaceous gland. Disorders associated with sebaceous glands include acne, seborrhea, sebaceoma, sebaceous carcinoma, seborrheic dermatitis, sebaceous cysts, sebaceous adenoma and sebaceous hyperplasia.

The term "acne" includes acne vulgaris and cystic acne and is characterized by areas of skin with seborrhea, comedones (blackheads and whiteheads), papules (pinheads), nodules (large papules) and pimples.

The term "seborrhea" includes oily skin.

The term "sebaceoma" includes benign tumors of the sebaceous gland. Sebaceoma may be induced by hereditary neoplasm cancer syndromes, such as Muir Torre syndrome.

The language "sebaceous carcinoma" includes malignant cutaneous tumors of the sebaceous glands, including extraocular and periocular sebaceous carcinomas.

The language "seborrheic dermatitis" includes inflammatory skin disorders characterized by scaly, flaky, itchy, and red skin and includes seborrheic dermatitis caused by fungal, genetic, environmental, hormonal and immune function disorders.

The language "sebaceous cysts" include steatocystoma simplex (*e.g*., simple sebaceous duct cyst and solitary steatocystoma and steatocystoma multiplex (*e.g*., epidermal polycystic disease and sebocystomatosis).

The language "sebaceous hyperplasia" includes enlargement of the sebaceous glands.

The term "subject" includes warm blooded mammals, for example, primates, cows, pigs, sheep, dogs, cats, rabbits, rats, and mice. In some embodiments, the subject is a primate, for example, a human. In some embodiment, the subject is suffering from polycystic ovary syndrome (*e.g*., POC). In some embodiments, the subject is suffering from acne. In some embodiments, the subject is suffering from seborrhea. In some embodiments, the subject is suffering from sebaceoma. In some embodiments, the subject is suffering from sebaceous carcinoma. In some embodiments, the subject is suffering form seborrheic dermatitis. In some embodiments, the subject is suffering from sebaceous cysts. In some embodiments, the subject is suffering from sebaceous adenoma. In some embodiments, the subject is suffering from sebaceous hyperplasia.

The language "effective amount" includes an amount of 2-((1*R*,4*R*)-4-(4-(6-carbamoyl-3,5-dimethylpyrazin-2-yl)phenyl)cyclohexyl)acetic acid , or a pharmaceutically acceptable salt thereof, that will elicit a biological or medical response in a subject, for example, the reduction or inhibition of enzyme or protein activity related to sebaceous gland function (for example, DGAT1), amelioration of symptoms of a disorder associated with sebaceous glands (*e.g*., acne, seborrhea, sebaceoma, sebaceous carcinoma, sebaceous dermatitis, sebaceous cysts, sebaceous adenoma and sebaceous hyperplasia) or the slowing or delaying of progression of a disorder associated with sebaceous glands. In some embodiments, the language "effective amount" includes the amount of 2-((1*R*,4*R*)-4-(4-(6-carbamoyl-3,5-dimethylpyrazin-2-yl)phenyl)cyclohexyl)acetic acid, or a pharmaceutically acceptable salt thereof, that when administered to a subject, is effective to at least partially alleviate, inhibit, and/or ameliorate a disorder associated with sebaceous glands in a subject.

The compound 2-((1*R*,4*R*)-4-(4-(6-carbamoyl-3,5-dimethylpyrazin-2-yl)phenyl)cyclohexyl)acetic acid refers to: 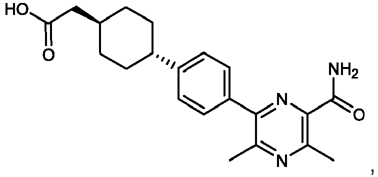 which was described in International Application Publication No. WO2009/081195.

The language "pharmaceutically acceptable salt" includes acid addition or base salts that retain the biological effectiveness and properties of the compounds of this invention and, which typically are not biologically or otherwise undesirable. In many cases, the compounds of the present invention are capable of forming acid and/or base salts by virtue of the presence of amino and/or carboxyl groups or groups similar thereto.

Pharmaceutically acceptable acid addition salts can be formed with inorganic acids and organic acids, *e.g*., acetate, aspartate, benzoate, besylate, bromide/hydrobromide, bicarbonate/carbonate, bisulfate/sulfate, camphorsulfonate, chloride/hydrochloride, chlortheophyllonate, citrate, ethandisulfonate, fumarate, gluceptate, gluconate, glucuronate, hippurate, hydroiodide/iodide, isethionate, lactate, lactobionate, laurylsulfate, malate, maleate, malonate, mandelate, mesylate, methylsulphate, naphthoate, napsylate, nicotinate, nitrate, octadecanoate, oleate, oxalate, palmitate, palmoate, phosphate/hydrogen phosphate/dihydrogen phosphate, polygalacturonate, propionate, stearate, succinate, subsalicylate, tartrate, tosylate and trifluoroacetate salts. Inorganic acids from which salts can be derived include, for example, hydrochloric acid, hydrobromic acid, sulfuric acid, nitric acid, phosphoric acid, and the like. Organic acids from which salts can be derived include, for example, acetic acid, propionic acid, glycolic acid, oxalic acid, maleic acid, malonic acid, succinic acid, fumaric acid, tartaric acid, citric acid, benzoic acid, mandelic acid, methanesulfonic acid, ethanesulfonic acid, toluenesulfonic acid, trifluoroacetic acid, sulfosalicylic acid, and the like. Pharmaceutically acceptable base addition salts can be formed with inorganic and organic bases.

Inorganic bases from which salts can be derived include, for example, ammonium salts and metals from columns I to XII of the periodic table. In certain embodiments, the salts are derived from sodium, potassium, ammonium, calcium, magnesium, iron, silver, zinc, and copper; particularly suitable salts include ammonium, potassium, sodium, calcium and magnesium salts. Organic bases from which salts can be derived include, for example, primary, secondary, and tertiary amines, substituted amines including naturally occurring substituted amines, cyclic amines, basic ion exchange resins, and the like. Certain organic amines include isopropylamine, benzathine, cholinate, diethanolamine, diethylamine, lysine, meglumine, piperazine and tromethamine.

The pharmaceutically acceptable salts of the present invention can be synthesized from a basic or acidic moiety, by conventional chemical methods. Generally, such salts can be prepared by reacting free acid forms of these compounds with a stoichiometric amount of the appropriate base (such as Na⁺, Ca²⁺, Mg²⁺, or K⁺ hydroxide, carbonate, bicarbonate or the like), or by reacting free base forms of these compounds with a stoichiometric amount of the appropriate acid. Such reactions are typically carried out in water or in an organic solvent, or in a mixture of the two. Generally, use of non-aqueous media like ether, ethyl acetate, ethanol, isopropanol, or acetonitrile is desirable, where practicable. Lists of additional suitable salts can be found, *e.g*., in "Remington's Pharmaceutical Sciences," 20th ed., Mack Publishing Company, Easton, Pa., (1985); and in "Handbook of Pharmaceutical Salts: Properties, Selection, and Use" by Stahl and Wermuth (Wiley-VCH, Weinheim, Germany, 2002).

The invention pertains, in a further aspect, to an effective amount of 2-((1*R*,4*R*)-4-(4-(6-carbamoyl-3,5-dimethylpyrazin-2-yl)phenyl)cyclohexyl)acetic acid, or a pharmaceutically acceptable salt thereof, for use in inducing sebaceous gland atrophy in a subject.

The term "inducing" includes an increase in the baseline activity of a biological activity or process.

The language "sebaceous gland atrophy" includes the partial or complete physiological wasting (*e.g*., reabsorption and breakdown of tissues of the sebaceous glands). In some embodiments, administration of 2-((1*R*,4*R*)-4-(4-(6-carbamoyl-3,5-dimethylpyrazin-2-yl)phenyl)cyclohexyl)acetic acid, or a pharmaceutically acceptable salt thereof, induces partial sebaceous gland atrophy. In some embodiments, administration of 2-((1*R*,4*R*)-4-(4-(6-carbamoyl-3,5-dimethylpyrazin-2-yl)phenyl)cyclohexyl)acetic acid, or a pharmaceutically acceptable salt thereof, induces complete sebaceous gland atrophy.

The invention also pertains, in a further aspect, to an effective amount of 2-((1*R*,4*R*)-4-(4-(6-carbamoyl-3,5-dimethylpyrazin-2-yl)phenyl)cyclohexyl)acetic acid, or a pharmaceutically acceptable salt thereof, for use in reducing sebum production in a subject.

The term "reducing" includes a decrease in the baseline activity of a biological activity or process.

The term "sebum" includes the secretions of the sebum glands and is composed of triglycerides, diglycerides, free fatty acids, wax esters, squalene and cholesterol. In some embodiments, reducing production of sebum includes reducing of one or more of the foregoing components of sebum. In one embodiment, administration of 2-((1*R*,4*R*)-4-(4-(6-carbamoyl-3,5-dimethylpyrazin-2-yl)phenyl)cyclohexyl)acetic acid, or a pharmaceutically acceptable salt thereof reduces the production of wax esters
The disorder associated with sebaceous glands may be acne, seborrhea, sebaceoma, sebaceous carcinoma, sebborheic dermatitis, sebborheic cysts, sebaceous adenoma and/or sebaceous hyperplasia.
The effective amount of 2-((1*R*,4*R*)-4-(4-(6-carbamoyl-3,5-dimethylpyrazin-2-yl)phenyl)cyclohexyl)acetic acid, or a pharmaceutically acceptable salt thereof, may be a pharmaceutical composition comprising 2-((1*R*,4*R*)-4-(4-(6-carbamoyl-3,5-dimethylpyrazin-2-yl)phenyl)cyclohexyl)acetic acid, or a pharmaceutically acceptable salt thereof and a pharmaceutically acceptable carrier, diluent or excipient.

The language "pharmaceutically acceptable excipient, carrier or diluent" includes compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The compositions of the invention may be in a form suitable for oral use (for example as tablets, lozenges, hard or soft capsules, aqueous or oily suspensions, emulsions, dispersible powders or granules, syrups or elixirs). The compositions of the invention may be obtained by conventional procedures using conventional pharmaceutical excipients well known in the art. Thus, compositions intended for oral use may contain, for example, one or more coloring, sweetening, flavoring and/or preservative agents.

Suitable pharmaceutically acceptable excipients for a tablet formulation include, for example, inert diluents such as lactose, sodium carbonate, calcium phosphate or calcium carbonate; granulating and disintegrating agents such as corn starch or algenic acid; binding agents such as starch; lubricating agents such as magnesium stearate, stearic acid or talc; preservative agents such as ethyl or propyl p-hydroxybenzoate; and anti-oxidants, such as ascorbic acid. Tablet formulations may be uncoated or coated either to modify their disintegration and the subsequent absorption of the active ingredient within the gastrointestinal tract, or to improve their stability and/or appearance, in either case, using conventional coating agents and procedures well known in the art.

Compositions for oral use may be in the form of hard gelatin capsules in which the active ingredient is mixed with an inert solid diluent, for example, calcium carbonate, calcium phosphate or kaolin, or as soft gelatin capsules in which the active ingredient is mixed with water or an oil such as peanut oil, liquid paraffin, or olive oil.

Aqueous suspensions generally contain the active ingredient in finely powdered form or in the form of nano or micronized particles together with one or more suspending agents, such as sodium carboxymethylcellulose, methylcellulose, hydroxypropylmethylcellulose, sodium alginate, polyvinyl-pyrrolidone, gum tragacanth and gum acacia; dispersing or wetting agents such as lecithin or condensation products of an alkylene oxide with fatty acids (for example polyoxethylene stearate), or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives such as ethyl or propyl p-hydroxybenzoate; anti-oxidants such as ascorbic acid); coloring agents; flavoring agents; and/or sweetening agents such as sucrose, saccharine or aspartame.

Oily suspensions may be formulated by suspending the active ingredient in a vegetable oil such as arachis oil, olive oil, sesame oil or coconut oil or in a mineral oil such as liquid paraffin. The oily suspensions may also contain a thickening agent such as beeswax, hard paraffin or cetyl alcohol. Sweetening agents such as those set out above, and flavoring agents may be added to provide a palatable oral preparation. These compositions may be preserved by the addition of an anti-oxidant such as ascorbic acid.

Dispersible powders and granules suitable for preparation of an aqueous suspension by the addition of water generally contain the active ingredient together with a dispersing or wetting agent, suspending agent and one or more preservatives. Suitable dispersing or wetting agents and suspending agents are exemplified by those already mentioned above. Additional excipients such as sweetening, flavoring and coloring agents, may also be present.

The pharmaceutical compositions of the invention may also be in the form of oil-in-water emulsions. The oily phase may be a vegetable oil, such as olive oil or arachis oil, or a mineral oil, such as for example liquid paraffin or a mixture of any of these. Suitable emulsifying agents may be, for example, naturally-occurring gums such as gum acacia or gum tragacanth, naturally-occurring phosphatides such as soya bean, lecithin, an esters or partial esters derived from fatty acids and hexitol anhydrides (for example sorbitan monooleate) and condensation products of the said partial esters with ethylene oxide such as polyoxyethylene sorbitan monooleate. The emulsions may also contain sweetening, flavoring and preservative agents.

Syrups and elixirs may be formulated with sweetening agents such as glycerol, propylene glycol, sorbitol, aspartame or sucrose, and may also contain a demulcent, preservative, flavoring and/or coloring agent.

The amount of active ingredient that is combined with one or more excipients to produce a single dosage form will necessarily vary depending upon the host treated and the particular route of administration. For further information on Routes of Administration and Dosage Regimes the reader is referred to Chapter 25.3 in Volume 5 of Comprehensive Medicinal Chemistry (Corwin Hansch; Chairman of Editorial Board), Pergamon Press 1990.

The invention pertains, to 2-((1*R*,4*R*)-4-(4-(6-carbamoyl-3,5-dimethylpyrazin-2-yl)phenyl)cyclohexyl)acetic acid, or a pharmaceutically acceptable salt thereof, for use in inducing sebaceous gland atrophy, for use in reducing sebum production or for use in treating acne, seborrhea, sebaceoma, sebaceous carcinoma, seborrheic dermatitis, seborrheic cysts, sebaceous adenoma and/or sebaceous hyperplasia.

In some embodiments, the invention pertains, at least in part, to a pharmaceutical composition comprising 2-((1*R*,4*R*)-4-(4-(6-carbamoyl-3,5-dimethylpyrazin-2-yl)phenyl)cyclohexyl)acetic acid, or a pharmaceutically acceptable salt thereof, for use in inducing sebaceous gland atrophy, for use in reducing sebum production or for use in treating acne, seborrhea, sebaceoma, sebaceous carcinoma, seborrheic dermatitis, seborrheic cysts, sebaceous adenoma and/or sebaceous hyperplasia.

In some embodiments, the invention pertains, at least in part, to a pharmaceutical composition comprising 2-((1*R*,4*R*)-4-(4-(6-carbamoyl-3,5-dimethylpyrazin-2-yl)phenyl)cyclohexyl)acetic acid, or a pharmaceutically acceptable salt thereof, and a pharmaceutically acceptable carrier, excipient and diluent, for use in inducing sebaceous gland atrophy, for use in reducing sebum production or for use in treating acne, seborrhea, sebaceoma, sebaceous carcinoma, seborrheic dermatitis, seborrheic cysts, sebaceous adenoma and/or sebaceous hyperplasia.

### Exemplification of the Invention

### MATERIALS AND METHODS

### Test Substance

2-((1*R*,4*R*)-4-(4-(6-carbamoyl-3,5-dimethylpyrazin-2-yl)phenyl)cyclohexyl)acetic acid was synthesised by AstraZeneca R&D, Alderley Park, UK and has been confirmed to show selective DGAT1 inhibitory activity (Barlind *et al.* 2012). In an *in vitro* recombinant mouse, dog, and human DGAT1 enzyme assay, 2-((1*R*,4*R*)-4-(4-(6-carbamoyl-3,5-dimethylpyrazin-2-yl)phenyl)cyclohexyl)acetic acid had an IC₅₀ of approximately 100, 60 and 80 nM, respectively and an IC₅₀ of >35000 nM against human ACAT1 and ACAT2 enzymes. No activity against human DGAT2 was detected.

For in vivo experiments, 2-((1*R*,4*R*)-4-(4-(6-carbamoyl-3,5-dimethylpyrazin-2-yl)phenyl)cyclohexyl)acetic acid was formulated as a suspension of up to 100 mg/ml in water containing HPMC/Tween (0.5% hydroxypropylmethycellulose + 0.1% polysorbate 80, Sigma-Aldrich, UK) which served as the vehicle control. Generally, 2-((1*R*,4*R*)-4-(4-(6-carbamoyl-3,5-dimethylpyrazin-2-yl)phenyl)cyclohexyl)acetic acid and the vehicle were formulated on the day prior to study start and remade every 7 days where necessary. The suspensions were stirred continuously from preparation throughout use and protected from light.

### Experimental Design

Doses and frequency of dosing were selected following consideration of species specific tolerability and pharmacokinetic parameters. Doses predicted to result in exposures which were likely to cause maximum DGAT1 inhibition without resulting in intolerability were chosen as the highest dose level. Animals were randomized into groups according to their body weight and dosed daily with 2-((1*R*,4*R*)-4-(4-(6-carbamoyl-3,5-dimethylpyrazin-2-yl)phenyl)cyclohexyl)acetic acid or vehicle, orally by gavage. Toxicokinetic sampling on the last day of dosing prior to necropsy was employed to assess the exposure profiles of animals given 2-((1*R*,4*R*)-4-(4-(6-carbamoyl-3,5-dimethylpyrazin-2-yl)phenyl)cyclohexyl)acetic acid.

### Experimental Procedures

Up to 32 (16 male and 16 female) mice (Crl:CD-1(ICR)) per dose group were obtained from the AstraZeneca breeding unit (Alderley Park, Macclesfield, UK). The animals, aged 7-8 weeks, were allowed to acclimatise for at least one week, were multiple-housed, sexes separately, up to 5/cage. Water from the site drinking water supply and RM1 (E) SQC pelleted diet supplied by Special Diets Services Ltd., England, was freely available. Nesting material (Tapvei Aspen Chips, Tapvei small aspen bricks, Finland, sizzle nests and paper shavings, and polycarbonate tunnels, Datesand,UK) was provided. Animals were terminated for scheduled necropsy on the day after the final dose. The mice were killed by administration of halothane. 2-((1*R*,4*R*)-4-(4-(6-carbamoyl-3,5-dimethylpyrazin-2-yl)phenyl)cyclohexyl)acetic acid was administered orally, once daily, to mice at 100, 300, and 600 mg/kg for 7 days, 0, 10, 80, and 300 mg/kg for 1 month, and 0, 1, 10, 60, and 300 mg/kg for up to 6 months.

In order to compare the effects of DGAT1 inhibition mediated by 2-((1*R*,4*R*)-4-(4-(6-carbamoyl-3,5-dimethylpyrazin-2-yl)phenyl)cyclohexyl)acetic acid on the skin with skin effects due to genetic DGAT1 deficiency, DGAT1 (-/-) mice were obtained from the AZ breeding unit (AstraZeneca Mölndal, Gothenburg, Sweden). To construct the DGAT1 gene targeting vector, 5.0 kb, 3.4 kb and 1.5 kb genomic DNA fragments were prepared from a BAC clone, which contained approximately 200 kb C57BI/6 genomic sequence including the Dgat1 gene (ResGen, Invitrogen Corporation), for 5' homology, deletion and 3' homology sequences respectively. The fragments were cloned into a loxP floxed PGKneo plasmid, using standard cloning techniques. In brief, a 3.3 kb fragment containing exons 3 to 17 of DGAT1 was inserted between an isolated loxP site and loxP-flanked PGK-Neomycin resistance cassette and then inserted between the two homology sequences. The DGAT1 targeting vector was linearized and electroporated into C57BI/6 (PRX-B6N #1, Primogenix) mouse embryonic stem (ES) cells and ES cell clones were selected with G418 (300 µg/ml). Candidate ES cell clones were screened by PCR and targeting was confirmed by Southern blot analysis using standard protocols. Targeted ES cells were injected into BALB/cAnNCrl blastocysts and injected embryos were implanted into the uteruses of pseudo-pregnant BALB/cAnNCrl mice. Chimeric males were mated with C57BI/6 females and resulting heterozygous F1 males were bred with Rosa26Cre transgenic mice on a C57BI/6 background to delete the loxP floxed region, including exons 3 to 17 in DGAT1, and the Neomycin cassette. DGAT1 null mice were obtained by heterozygote intercrossing and the mutations were confirmed by RT-PCR analysis. Animals were kept in pathogen free environment and routinely screened for pathogens.

Prior to the animal studies mice, aged 6-12 weeks, were allowed to acclimatise for at least one week, were multiple-housed, sexes separately, up to 5/cage. Water from the site drinking water supply and RM1 (E) SQC pelleted diet supplied by Special Diets Services Ltd., England, was freely available. Nesting material (Tapvei Aspen Chips, Tapvei small aspen bricks, Finland, sizzle nests and paper shavings, and polycarbonate tunnels, Datesand,UK) was provided. Animals were killed by administration of halothane.

Up to 8 (4 male and 4 female) beagle dogs per treatment group were obtained from the AstraZeneca breeding unit (Alderley Park, Macclesfield, UK). The animals, aged 12 to 24 months, were allowed to acclimatise for at least one week, were multiply housed and given ad libitum access to water and standard dog diet (Diet 125 C3, Safe, France). 2-((1*R*,4*R*)-4-(4-(6-carbamoyl-3,5-dimethylpyrazin-2-yl)phenyl)cyclohexyl)acetic acid was administered orally, once daily, to dogs at 100 and 1000 mg/kg/day for 7 days, and twice daily at 0, 20, 160, and 2000 mg/kg/day for 14 days, 0, 4, 20, 140, and 500 mg/kg/day for up to 3 months, and 0, 4, 16, and 100 mg/kg/day for up to 6 months. All animals were observed daily for clinical signs of toxicity. Body weight and food consumption was measured daily prior to dosing. Animals were single-penned, with access to an exercise area with others of the same sex and group, for the major part of each day. Animals had free access to water from the site drinking water supply. A set weight of SDS Dog-D3 (E) SQC, supplied by Special Diet Services Ltd, England, was provided daily, in the afternoon, access being allowed for up to approximately 2 hours. Toys (e.g., bones) were provided for additional environmental enhancement. Animals were terminated for scheduled necropsy on the day after the final dose. The dogs were killed by intravenous administration of sodium pentobarbitone and then exsanguinated.

All studies were conducted in strict adherence to the UK Home Office regulations for animal welfare (Animal Scientific Procedures 1986 Act).

For pharmacokinetic analysis, blood samples were collected into lithium heparin tubes from animals on either the first or penultimate day of the study. Plasma was separated by centrifugation at 4°C and stored for analysis at -20°C. Plasma concentrations of 2-((1*R*,4*R*)-4-(4-(6-carbamoyl-3,5-dimethylpyrazin-2-yl)phenyl)cyclohexyl)acetic acid were determined by liquid chromatography-mass spectrometry (LC-MS) and pharmacokinetic parameters of maximum plasma drug concentration (Cₘₐₓ) and concentration at the dose interval (Cₘᵢₙ) calculated. Exposure data are presented as FDM (free drug concentration in blood plasma required to inhibit the microsomal DGAT1 enzyme by 50%) and are mean data for each dose level. Due to a significant (∼1.5 - 3x) sex difference in mice, separate data points are given for male and female mice. There was no sex difference in dogs and data points are mean data of males and females combined.
At the end of each experiment, animals were terminally anaesthetised and representative samples of skin (mammary gland region for mice, abdominal region for dogs) were taken at necropsy, placed in 10% buffered formalin and processed for histopathological assessment. Conventional processing into wax and haematoxylin and eosin staining of sections preceded their light microscopic examination.

Light microscopic examination and histology scoring was performed by a certified pathologist and findings were peer reviewed by a second pathologist. Each section of skin contained from 15 - 25 hair follicles in different stages of differentiation with associated sebaceous glands that were cut in multiple planes. There was no difference in sebaceous gland appearances within or between tissue sections from control animals, *i.e.,* there was no apparent alteration in sebaceous cell morphology related to stage of hair follicle differentiation. Therefore, assessment of sebaceous cell/gland atrophy was based on their appearances irrespective of the stage of differentiation of the associated hair follicle in all instances. Severity scores ranged from no change by comparison with controls, to severe, which was defined as the total absence of sebaceous glands in all skin tissue sections examined from an individual animal. Minimal severity was defined as sebaceous cell numbers per gland being as that seen in controls but cell size was uniformly reduced, mild as a small reduction in sebaceous cell numbers but with substantially reduced (and variable) cell size, and moderate, as very few cells remaining that appeared shrunken.

### Effects of DGAT1 inhibition in the mouse

2-((1*R*,4*R*)-4-(4-(6-carbamoyl-3,5-dimethylpyrazin-2-yl)phenyl)cyclohexyl)acetic acid was administered to mice, orally, once daily, for 7, 28 or 180 days at doses predicted to cause minimal to substantial DGAT1 inhibition over the dose interval. Toxicokinetic analysis revealed that Cₘₐₓ was above the mouse microsomal DGAT1 enzyme IC₅₀ at all dose levels, ranging from 4x IC₅₀ in the 1mg/kg/day dose group in the 180 day study to 1581x IC₅₀ in the 600 mg/kg/day dose group in the 7 day study. For all dose levels and durations Cₘᵢₙ was above the DGAT1 IC₅₀ for the duration of the dose interval, ranging from just 1x IC₅₀ in the 1mg/kg/day dose group in the 180 day study to 766x IC₅₀ in the 600 mg/kg/day dose group in the 7 day study, respectively.

In the mouse, administration of 2-((1*R*,4*R*)-4-(4-(6-carbamoyl-3,5-dimethylpyrazin-2-yl)phenyl)cyclohexyl)acetic acid was well tolerated in all studies. Significant drug-induced sebaceous gland atrophy in the skin characterised by a reduction in size of sebaceous glands attached to each hair follicle was detected upon histopathological examination of skin samples (**Figure 1**). Histopathological data from the 1 month and 6 month studies indicated that sebaceous gland atrophy did not occur when group mean exposures were ≤ 6x FDM at Cₘₐₓ and 1.5x at Cₘᵢₙ during the dose interval. Sebaceous gland atrophy of minimal severity occurred at mean exposures of 22x FDM at Cₘₐₓ and 10x Cₘᵢₙ, indicating the necessity of a high level of continuous DGAT1 enzyme inhibition for this morphological change to occur. At higher exposures minimal to mild sebaceous gland atrophy was noted at 50 - 260x FDM at Cₘₐₓ and 7 - 40x FDM at Cₘᵢₙ and mild to moderate sebaceous gland atrophy was noted at an exposure of 226x FDM at Cₘₐₓ and 46x FDM at Cₘᵢₙ. The relationship between FDM (Cₘₐₓ and Cₘᵢₙ) during the dose interval and severity of sebaceous gland atrophy is shown in **Figure 2****.**

In addition to a relationship between 2-((1*R*,4*R*)-4-(4-(6-carbamoyl-3,5-dimethylpyrazin-2-yl)phenyl)cyclohexyl)acetic acid exposure and level of sebaceous gland atrophy, an association between duration of exposure and level of this finding was observed. Whilst sebaceous gland atrophy was absent in mice treated with high doses of 2-((1*R*,4*R*)-4-(4-(6-carbamoyl-3,5-dimethylpyrazin-2-yl)phenyl)cyclohexyl)acetic acid for up to 7 days, the group mean severity of this finding ranged from minimal to mild after 1 month (28 days) of dosing which increased to minimal to moderate severity after 6 months (180 days) of dosing (**Figure 3**). Following a 4 week drug-free period the observed sebaceous gland atrophy noted after 1 month of treatment had resolved, indicating complete reversibility of this finding (**Figure 1**). However, reversibility was not assessed following 6 months of dosing.

Whilst histopathological findings in the skin, following administration of AZD7687 for 1 month, were restricted to sebaceous gland atrophy only, after dosing for 6 months, this finding was also associated with epidermal hyperplasia, occasional hair follicle atrophy, as well as in-life observation of hair-loss and skin lesions (*i.e*., open wounds and scabs). These additional skin findings occurred at exposure levels of at least 50x FDM at Cₘₐₓ and 7x FDM at Cₘᵢₙ, levels which were sufficient to cause substantial DGAT1 inhibition during the dose interval and were noted to be more significant at higher levels of sebaceous gland atrophy (**Figure 4**). There were no notable sex differences, the only exception being the increased incidence in skin lesions observed in-life which was higher in males than in females. The histopathological phenotype observed following substantial DGAT1 inhibition for 6 months closely resembled that of DGAT1 knockout mice. **Figure 5** shows skin photomicrographs of male wildtype and DGAT1(-/-) mice as well as male mice treated with vehicle or 2-((1*R*,4*R*)-4-(4-(6-carbamoyl-3,5-dimethylpyrazin-2-yl)phenyl)cyclohexyl)acetic acid for 6 months.

### Effects of DGAT1 inhibition in the dog

2-((1*R*,4*R*)-4-(4-(6-carbamoyl-3,5-dimethylpyrazin-2-yl)phenyl)cyclohexyl)acetic acid was administered to dogs, orally, once daily for 7, and twice daily for 28 or 180 days at doses predicted to cause minimal to marked DGAT1 inhibition over the dose interval. Toxicokinetic analysis revealed that Cₘₐₓ was above the dog microsomal DGAT1 enzyme IC₅₀ at all dose levels, ranging from 10x IC₅₀ in the 4 mg/kg/day dose group in the 3 month study to 292x IC₅₀ in the 1000 mg/kg/day dose group in the same study. For all dose levels and durations, Cₘᵢₙ was above the DGAT1 IC₅₀ for the duration of the dose interval, ranging from just 1x IC₅₀ in the 4 mg/kg/day dose group in the 3 months study to 54x IC₅₀ in the 1000 mg/kg/day dose group in the 7 day study.

Administration of 2-((1*R*,4*R*)-4-(4-(6-carbamoyl-3,5-dimethylpyrazin-2-yl)phenyl)cyclohexyl)acetic acid was well tolerated in all studies. As in the mouse, drug-induced sebaceous gland atrophy in the skin was detected following histopathological examination (**Figure 6**) and a clear dose-response relationship was noted. Thus, group mean exposures of up to 12x FDM at Cₘₐₓ and 2x at Cₘᵢₙ during the dose interval did not result in sebaceous gland atrophy. However, minimal sebaceous gland atrophy occurred at mean exposures of 32x FDM at Cₘₐₓ and 7x FDM at Cₘᵢₙ, indicating that this level of continuous DGAT1 enzyme inhibition was required for this morphological change to occur. At higher exposures, minimal to mild sebaceous gland atrophy was noted at 48-193x FDM at Cₘₐₓ and 7-54x FDM at Cₘᵢₙ and mild to moderate sebaceous gland atrophy noted at an exposure above 165x FDM at Cₘₐₓ and 36x FDM at Cₘᵢₙ. The relationship between FDM (Cₘₐₓ and Cₘᵢₙ) and severity of sebaceous gland atrophy is shown in **Figure 7****.**

A clear relationship to duration of exposure and severity of sebaceous gland atrophy was also evident. Whilst sebaceous gland atrophy was absent in dogs dosed for up to 7 days, the group mean severity of this finding ranged from minimal to mild after 14 days of dosing with an increase to minimal to moderate mean severity after 3 months (90 days) of dosing and further increase to moderate level in the highest dose group after 6 months (180 days) (**Figure 8**). Following a 4 week drug-free period, the observed sebaceous gland atrophy noted after 3 months of dosing had resolved, indicating complete reversibility of this finding. Reversibility was not assessed following 6 months of dosing.

In addition to sebaceous gland atrophy, minimal hyperkeratosis of the epithelium covering the infundibula of hair follicles was observed in the dog following exposure to 2-((1*R*,4*R*)-4-(4-(6-carbamoyl-3,5-dimethylpyrazin-2-yl)phenyl)cyclohexyl)acetic acid for 6 months. However, the additional skin changes observed after 6 months of treatment in the mouse (*i.e.,* occasional hair follicle atrophy, as well as in-life observation of hair-loss and skin lesions) were not observed in the dog.

Following administration of 2-((1*R*,4*R*)-4-(4-(6-carbamoyl-3,5-dimethylpyrazin-2-yl)phenyl)cyclohexyl)acetic acid, sebaceous gland atrophy was noted at Cₘₐₓ/Cₘᵢₙ levels of at least 22x/10x and 32x/7x FDM, but was absent at 6x/1.5x and 12x/2x times the FDM in the mouse and the dog, respectively. This is indicative of the requirement for substantial levels of DGAT1 inhibition, sustained throughout the dosing interval, for induction of sebaceous gland atrophy. Furthermore, with regard to duration of exposure in both species, inhibition of DGAT1 by 2-((1*R*,4*R*)-4-(4-(6-carbamoyl-3,5-dimethylpyrazin-2-yl)phenyl)cyclohexyl)acetic acid for more than 7 days was required for microscopical detection of sebaceous gland atrophy, demonstrating that not only substantial, but also prolonged inhibition of DGAT1 was a key requirement.

In the mouse, but not in the dog, inhibition of DGAT1 for 6 months also resulted in significant epidermal hyperplasia, occasional hair follicle atrophy, as well as in-life observation of hair-loss and skin lesions. This phenotype, which was only seen at high levels of DGAT1 inhibition, was similar to that seen in the DGAT1 (-/-) mouse that exhibit dry fur and hair-loss and which is associated with atrophic sebaceous glands and fur lipid abnormalities, especially the absence of wax esters (Chen *et al.* 2002; Yen *et al.* 2005). Fur and sebaceous gland abnormalities were most apparent in post pubertal male DGAT1 (-/-) mice, suggesting a potential hormone-based sex difference (Chen *et al.* 2002). Our data did not indicate a gender difference in sebaceous gland atrophy, epidermal hyperplasia or hyperkeratosis, or in-life hair loss following administration of 2-((1*R*,4*R*)-4-(4-(6-carbamoyl-3,5-dimethylpyrazin-2-yl)phenyl)cyclohexyl)acetic acid. However, the increased incidence of in-life skin lesions was more pronounced in male animals and may indicate some role of androgens in mediating the effects of DGAT1 inhibition on the skin, although it cannot be excluded that fighting, which is commonly observed in group housed males was a significant influence.

These observations in the mouse suggest that DGAT1-mediated sebum production plays an important role in the skin and in hair development and their maintenance. In the dog, we observed sebaceous gland atrophy and minimal hyperkeratosis of the epithelium only, but not the more severe phenotype which included hair loss, dry fur, and skin lesions observed in the mouse, which implies that a species difference in the role of DGAT1 in fur maintenance of the mouse and the dog may exist. Whilst pharmacological inhibition of DGAT1 in the mouse indicates a potential for alopecia, this is not supported by our data in the dog and given the differences between human and animal skin surface lipids and the role of the eccrine sweating system in man, the effects of prolonged DGAT1 inhibition on the integrity of the human skin and scalp may be less significant. Interestingly, whilst sebaceous gland, sebaceous gland duct, or hair follicle inflammation is a common finding in human asebia (Al-Zaid et al. 2011) a significant increase in inflammation was not seen in the skin of mice and dogs treated with 2-((1*R*,4*R*)-4-(4-(6-carbamoyl-3,5-dimethylpyrazin-2-yl)phenyl)cyclohexyl)acetic acid. Folliculitis involving the sebaceous glands was very rare in the studies and post-inflammation scarring was not observed.

This data indicates that not all species show the same degree of adverse skin changes as a consequence of DGAT1 inhibition, and that these adverse changes only occur at sustained and substantial levels of DGAT1 inhibition or in DGAT1 deficient mice. To date, no effects of 2-((1*R*,4*R*)-4-(4-(6-carbamoyl-3,5-dimethylpyrazin-2-yl)phenyl)cyclohexyl)acetic acid administration on the skin of healthy volunteers have been noted in clinical trials of short duration (Denison *et al.* 2013). It is noteworthy that our data are also supportive of the use of pharmacological inhibition of DGAT1 in the treatment of seborrheic conditions such as acne, where a DGAT1 inhibitor-mediated atrophy of the sebaceous gland may result in a clinically relevant reduction in sebum production.

## Claims

1. An effective amount of 2-((1R,4R)-4-(4-(6-carbamoyl-3,5-dimethylpyrazin-2-yl)phenyl)cyclohexyl)acetic acid, or a pharmaceutically acceptable salt thereof for use in treating a disorder associated with sebaceous glands in a subject.

2. The effective amount of 2-((1R,4R)-4-(4-(6-carbamoyl-3,5-dimethylpyrazin-2-yl)phenyl)cyclohexyl)acetic acid, or a pharmaceutically acceptable salt thereof for the use of claim 1, wherein the disorder is selected from acne, seborrhea, sebaceoma, sebaceous carcinoma, sebborheic dermatitis, sebborheic cysts, sebaceous adenoma and sebaceous hyperplasia.

3. An effective amount of 2-((1R,4R)-4-(4-(6-carbamoyl-3,5-dimethylpyrazin-2-yl)phenyl)cyclohexyl)acetic acid, or a pharmaceutically acceptable salt thereof for use in inducing sebaceous gland atrophy in a subject.

4. The effective amount of 2-((1R,4R)-4-(4-(6-carbamoyl-3,5-dimethylpyrazin-2-yl)phenyl)cyclohexyl)acetic acid, or a pharmaceutically acceptable salt thereof for the use of claim 3, wherein the atrophy is partial or complete atrophy.

5. An effective amount of 2-((1R,4R)-4-(4-(6-carbamoyl-3,5-dimethylpyrazin-2-yl)phenyl)cyclohexyl)acetic acid, or a pharmaceutically acceptable salt thereof for use in reducing sebum production in a subject.

6. The effective amount of 2-((1R,4R)-4-(4-(6-carbamoyl-3,5-dimethylpyrazin-2-yl)phenyl)cyclohexyl)acetic acid, or a pharmaceutically acceptable salt thereof for the use of any one of claims 1-5, wherein the subject is a warm blooded animal.

7. The effective amount of 2-((1R,4R)-4-(4-(6-carbamoyl-3,5-dimethylpyrazin-2-yl)phenyl)cyclohexyl)acetic acid, or a pharmaceutically acceptable salt thereof for the use- of claim 6, wherein the warm blooded animal is human.

8. The effective amount of 2-((1R,4R)-4-(4-(6-carbamoyl-3,5-dimethylpyrazin-2-yl)phenyl)cyclohexyl)acetic acid, or a pharmaceutically acceptable salt thereof for the use of any one of claims 1-7, wherein the effective amount is about 300 miligrams/killigram/day.

9. The effective amount of 2-((1R,4R)-4-(4-(6-carbamoyl-3,5-dimethylpyrazin-2-yl)phenyl)cyclohexyl)acetic acid, or a pharmaceutically acceptable salt thereof for the use of any one of claims 1-8, wherein the medicament is for oral administration to the subject.

10. The effective amount of 2-((1R,4R)-4-(4-(6-carbamoyl-3,5-dimethylpyrazin-2-yl)phenyl)cyclohexyl)acetic acid, or a pharmaceutically acceptable salt thereof for the use of any one of claims 1-9, wherein the medicament is in a form selected from the group consisting of tablet, capsule, suspension, emulsion, syrup, and elixir.

## Patentansprüche

1. Wirksame Menge 2-((1R,4R)-4-(4-(6-Carbamoyl-3,5-dimethylpyrazin-2-yl)phenyl)cyclohexyl)essigsäure oder eines pharmazeutisch verträglichen Salzes davon zur Verwendung beim Behandeln einer Störung, die mit Talgdrüsen verbunden ist, bei einem Subjekt.

2. Wirksame Menge 2-((lR,4R)-4-(4-(6-Carbamoyl-3,5-dimethylpyrazin-2-yl)phenyl)cyclohexyl)essigsäure oder eines pharmazeutisch verträglichen Salzes davon zu der Verwendung nach Anspruch 1, wobei die Störung aus Akne, Seborrhoe, Sebazeom, Talgdrüsenkarzinom, seborrhoischer Dermatitis, seborrhoischen Zysten, Talgdrüsenadenom und Talgdrüsenhyperplasie ausgewählt ist.

3. Wirksame Menge 2-((1R,4R)-4-(4-(6-Carbamoyl-3,5-dimethylpyrazin-2-yl)phenyl)cyclohexyl)essigsäure oder eines pharmazeutisch verträglichen Salzes davon zur Verwendung beim Induzieren einer Talgdrüsenatrophie bei einem Subjekt.

4. Wirksame Menge 2-((lR,4R)-4-(4-(6-Carbamoyl-3,5-dimethylpyrazin-2-yl)phenyl)cyclohexyl)essigsäure oder eines pharmazeutisch verträglichen Salzes davon zu der Verwendung nach Anspruch 3, wobei die Atrophie eine teilweise oder vollständige Atrophie ist.

5. Wirksame Menge 2-((1R,4R)-4-(4-(6-Carbamoyl-3,5-dimethylpyrazin-2-yl)phenyl)cyclohexyl)essigsäure oder eines pharmazeutisch verträglichen Salzes davon zur Verwendung beim Verringern der Talgproduktion bei einem Subjekt.

6. Wirksame Menge 2-((lR,4R)-4-(4-(6-Carbamoyl-3,5-dimethylpyrazin-2-yl)phenyl)cyclohexyl)essigsäure oder eines pharmazeutisch verträglichen Salzes davon zu der Verwendung nach einem der Ansprüche 1 - 5, wobei das Subjekt ein warmblütiges Tier ist.

7. Wirksame Menge 2-((1R,4R)-4-(4-(6-Carbamoyl-3,5-dimethylpyrazin-2-yl)phenyl)cyclohexyl)essigsäure oder eines pharmazeutisch verträglichen Salzes davon zu der Verwendung nach Anspruch 6, wobei das warmblütige Tier ein Mensch ist.

8. Wirksame Menge 2-((1R,4R)-4-(4-(6-Carbamoyl-3,5-dimethylpyrazin-2-yl)phenyl)cyclohexyl)essigsäure oder eines pharmazeutisch verträglichen Salzes davon zu der Verwendung nach einem der Ansprüche 1 - 7, wobei die wirksame Menge etwa 300 Miligramm/Killigramm/Tag beträgt.

9. Wirksame Menge 2-((1R,4R)-4-(4-(6-Carbamoyl-3,5-dimethylpyrazin-2-yl)phenyl)cyclohexyl)essigsäure oder eines pharmazeutisch verträglichen Salzes davon zu der Verwendung nach einem der Ansprüche 1 - 8, wobei das Medikament zur oralen Verabreichung an das Subjekt bestimmt ist.

10. Wirksame Menge 2-((1R,4R)-4-(4-(6-Carbamoyl-3,5-dimethylpyrazin-2-yl)phenyl)cyclohexyl)essigsäure oder eines pharmazeutisch verträglichen Salzes davon zu der Verwendung nach einem der Ansprüche 1 - 9, wobei das Medikament in einer Form vorliegt, die aus der Gruppe bestehend aus Tablette, Kapsel, Suspension, Emulsion, Sirup und Elixier ausgewählt ist.

## Revendications

1. Quantité efficace d'acide 2-((1R,4R)-4-(4-(6-carbamoyl-3,5-diméthylpyrazin-2-yl) phényl)cyclohexyl)acétique, ou d'un sel pharmaceutiquement acceptable de celui-ci destinée à être utilisée dans le traitement d'un trouble associé aux glandes sébacées chez un sujet.

2. Quantité efficace d'acide 2-((1R,4R)-4-(4-(6-carbamoyl-3,5-diméthylpyrazin-2-yl) phényl)cyclohexyl)acétique, ou d'un sel pharmaceutiquement acceptable de celui-ci destinée à être utilisée selon la revendication 1, dans laquelle le trouble est choisi parmi l'acné, la séborrhée, le sébacéome, l'adénome sébacé, la dermatite séborrhéique, les kystes séborrhéiques, l'adénome sébacé et l'hyperplasie sébacée.

3. Quantité efficace d'acide 2-((1R,4R)-4-(4-(6-carbamoyl-3,5-diméthylpyrazin-2-yl) phényl)cyclohexyl)acétique, ou d'un sel pharmaceutiquement acceptable de celui-ci destinée à être utilisée dans l'induction d'une atrophie des glandes sébacées chez un sujet.

4. Quantité efficace d'acide 2-((1R,4R)-4-(4-(6-carbamoyl-3,5-diméthylpyrazin-2-yl) phényl)cyclohexyl)acétique, ou d'un sel pharmaceutiquement acceptable de celui-ci destinée à être utilisée selon la revendication 3, dans laquelle l'atrophie est une atrophie partielle ou totale.

5. Quantité efficace d'acide 2-((1R,4R)-4-(4-(6-carbamoyl-3,5-diméthylpyrazin-2-yl) phényl)cyclohexyl)acétique, ou d'un sel pharmaceutiquement acceptable de celui-ci destinée à être utilisée dans la réduction de la production de sébum chez un sujet.

6. Quantité efficace d'acide 2-((1R,4R)-4-(4-(6-carbamoyl-3,5-diméthylpyrazin-2-yl) phényl)cyclohexyl)acétique, ou d'un sel pharmaceutiquement acceptable de celui-ci destinée à être utilisée selon l'une quelconque des revendications 1 à 5, dans laquelle le sujet est un animal à sang chaud.

7. Quantité efficace d'acide 2-((1R,4R)-4-(4-(6-carbamoyl-3,5-diméthylpyrazin-2-yl) phényl)cyclohexyl)acétique, ou d'un sel pharmaceutiquement acceptable de celui-ci destinée à être utilisée selon la revendication 6, dans laquelle l'animal à sang chaud est un humain.

8. Quantité efficace d'acide 2-((1R,4R)-4-(4-(6-carbamoyl-3,5-diméthylpyrazin-2-yl) phényl)cyclohexyl)acétique, ou d'un sel pharmaceutiquement acceptable de celui-ci destinée à être utilisée selon l'une quelconque des revendications 1 à 7, dans laquelle la quantité efficace est d'environ 300 miligrammes/kiligramme/jour.

9. Quantité efficace d'acide 2-((1R,4R)-4-(4-(6-carbamoyl-3,5-diméthylpyrazin-2-yl) phényl)cyclohexyl)acétique, ou d'un sel pharmaceutiquement acceptable de celui-ci destinée à être utilisée selon l'une quelconque des revendications 1 à 8, dans laquelle le médicament est destiné à une administration par voie orale au sujet.

10. Quantité efficace d'acide 2-((1R,4R)-4-(4-(6-carbamoyl-3,5-diméthylpyrazin-2-yl) phényl)cyclohexyl)acétique, ou d'un sel pharmaceutiquement acceptable de celui-ci destinée à être utilisée selon l'une quelconque des revendications 1 à 9, dans laquelle le médicament est sous une forme choisie parmi le groupe constitué par un comprimé, une capsule, une suspension, une émulsion, un sirop, et un élixir.
